# EUROPEAN PATENT SPECIFICATION

(11) **EP 1 267 857 B1**
(45) Date of publication and mention of the grant of the patent: **14.02.2007**
(21) Application number: 01916022.5
(22) Date of filing: 21.03.2001
(51) Int. Cl.: A61K 31/12, C07C 403/24

(54) **In vitro use of astaxanthin for pretreatment of cells prior to UV-exposure to inhibit the activation of NF-kB**
In-vitro-Verwendung von Astaxanthin zur Vorbehandlung von Zellen vor UV-Bestrahlung zwecks Hemmung der Aktivierung von NF-KB
Utilisation in vitro d'astaxanthine pour le pretraitement de cellules avant exposition aux rayons UV afin d'inhiber l'activation de NF-KB

(30) Priority: 27.03.2000 SE 0001071
(43) Date of publication of application: 02.01.2003
(73) Proprietor: ASTACAROTENE AB, 134 40 Gustavsberg (SE)
(72) Inventor: ANDERSSON, Tove, 117 38 Stockholm (SE); PETTERSSON, Sven, 146 40 Tullinge (SE)
(74) Representative: Onn, Thorsten
(86) International application number: PCT/SE2001/000600
(87) International publication number: WO 2001/072296

(56) References cited:
- EP-A1- 0 770 385
- WO-A1-98/37874
- WO-A1-99/11251
- WO-A2-98/45241
- US-A- 5 527 533
- US-A- 5 886 053
- MICHI KURASHIGE ET AL.: 'Inhibition of oxidative injury of biological membranes by astaxanthin' PHYSIOL. CHEM. PHYS. & MED. NMR vol. 22, 1990, pages 27 - 38, XP002945512
- DATABASE CAPLUS [Online] DOC. NO. 104:85415 POPOVA N.V. ET AL.: 'Carotenoids of the fruit of capsicum annuum', XP002945513 Retrieved from STN Database accession no. 1986:85415 & FARM. ZH. vol. 6, 1985, KIEV, pages 50 - 54, XP002945514
- PATENT ABSTRACTS OF JAPAN & JP 07 099 924 A (NIPPON SUISAN KAISHA LTD) 18 April 1995
- PATENT ABSTRACTS OF JAPAN & JP 07 300 421 A (ITANO REITOU KK) 14 November 1995

## Description

The present invention relates to the in vitro use of astaxanthin for pretreatment of cells prior to UV-exposure to inhibit the activation of NF-κB.

### Background

The nuclear factor-κB (NF-κB) is a conditionally regulated transcription factor that plays a key role in the expression of a variety of genes involved in inflammation, cell survival, apoptosis, cell differentiation and cancer. It was first identified as a regulator of κ light chain expression in murine B-lymphocytes, but has now been shown to be expressed ubiquitously and to be a master regulator of several important processes. The NF-κB family consists of structurally related proteins of the Rel family, including p50, p52, p65/Re1A, c-Rel and RelB (reviewed in Rothwarf and Karin, 1999). In unstimulated cells, NF-κB is bound to the inhibitor protein IκB, which masks the nuclear localisation signal of NF-κB and retains it in the cytoplasm. Activation of the cell with various stimuli initiates signalling pathways involving activation of a whole series of protein kinases. This results in phosphorylation of IκB, targeting the protein for degradation (Rothwarf and Karin, 1999). As a result, the IκB/NF-κB complex dissociates, NF-κB translocates to the nucleus and binds to its cognate sites. Nuclear translocation of NF-κB is activated by various stimuli, including the inflammatory cytokines TNF-α and IL-1, UV-irradiation, mitogens, viruses, bacterias, double stranded DNA, ionizing radiation and hydrogen peroxide, in accordance with the important role played by NF-κB in various tissues (Rothwarf and Karin, 1999).

The functional importance of NF-κB in acute and chronic inflammation is based on its ability to regulate the promoters of a variety of genes. The products of such genes are e.g. cytokines, adhesion molecules and acute phase proteins, which are critical for inflammatory processes (Baeuerle *et al*., 1995, Shakov *et al*., 1990, Libermann *et al*., 1990). These findings are further underlined by the demonstration that mice containing targeted disruptions of the NF-κB subunits p50, RelB and c-Rel are compromised in various aspects of immune function and inflammatory processes (Sha *et al*., 1995; Weih *et al*., 1995, Köntgen *et al*.; 1995). Moreover, elevated levels of p65 have been observed in patients with rheumathoid arthritis and Inflammatory Bowel Disease (IBD). A role for NF-κB in inflammation was further established in a recent study, demonstrating that local administration of an antisense oligonucleotide targeted against the translational start site of NF-κB p65 abrogates established intestinal inflammation in mice (Neurath *et al*., 1996).

During the last years, it has become evident that redox regulation is an important mechanism that regulates conditional gene expression. Several transcription factors have been shown to be redox-regulated, including NF-κB. One common step in the activation mechanisms that lead to NF-κB translocation has been suggested to involve reactive oxygen species, based on the finding that NF-κB activation can be inhibited by a series of antioxidants (reviewed in Pitette *et al.,* 1997). However, little is known about the pathways that activate and control NF-κB e.g. during oxidative stress.

Due to the involvement of the transcription factor NF-κB in inflammatory processes it could be possible to inhibit the expression of inflammatory cytokines and chemokines by affecting the function of the NF-κB in an animal or human.

There are several reports, patents and patent applications that describe the usefulness of xanthophylls as antioxidants and/or anti-inflammatory agents in different contexts, see e.g. WO-A1-9837874; "Inhibition of oxidative injury of biological membranes by astaxanthin", MICHI KURASHIGE ET AL.","PHYSIOL. CHEM. PHYS. & MED. NMR, 22//00-00-1990, 27-23; "Carotenoids of fruit of capsicum annuum", POPOVA N.V. ET AL, CAPLUS, DOC. NO. 104:85415; WO-A2-9845241; US-A-5,527,533; PAJ Abstract of JP07-099924-A, 18-04-1995; PAJ Abstract of JP07-300421-A, 14-11-1995; WO-A1-9911251; EP-A1-0770385; and US-A-5,886,053. Further, WO O1/24787 describes the use of xanthophylls for suppression of excessive Th1 cell mediated responses and stimulation of Th2 cell mediated responses during ongoing infection and/or inflammation.

### Description of the invention

The present invention is directed to the in vitro use of astaxanthin for pretreatment of cells prior to UV-exposure to inhibit the activation of NF-κB.

Astaxanthin from other natural sources than algae, such as from fungi and crustaceans, are expected to be similarly useful for the purposes of the invention. An advantage of using astaxanthin from algae may be that the astaxanthin exists in a form esterified with fatty acids, which esterified astaxanthin thereby is more stable during handling and storage than free astaxanthin.

In a preferred embodiment the astaxanthin may be selected from the group consisting of astaxanthin from a natural source, such as a culture of the alga *Haematococcus sp*., synthetic astaxanthin and mixtures thereof.

### Description of the drawing

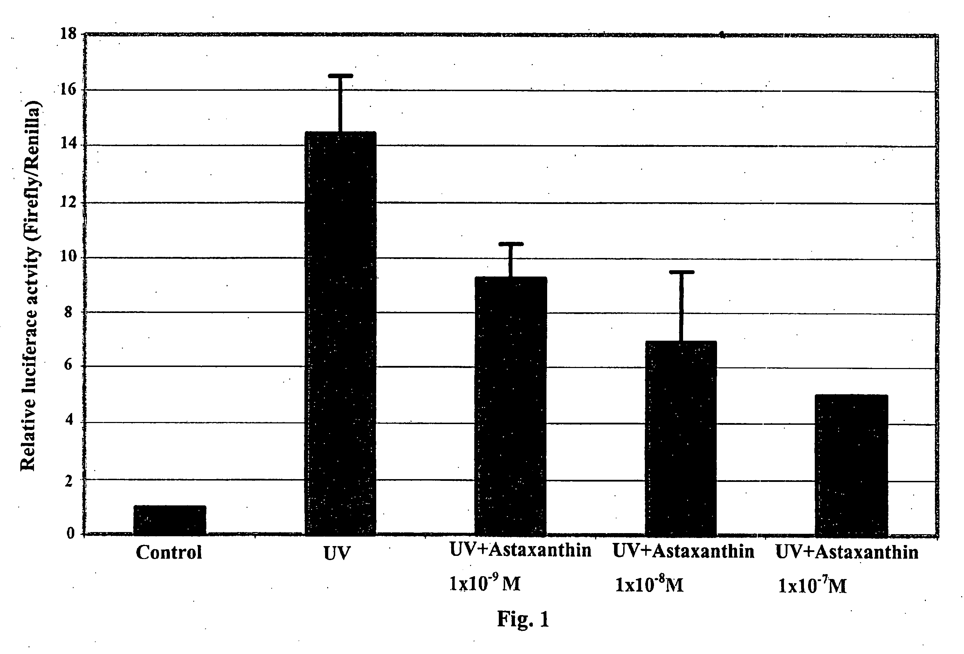

**Fig 1** is a diagram which shows that Astaxanthin inhibits the activation of NF-κB effected by UV exposure. HeLa cells were transfected with a NF-κB-dependent reporter gene and exposed to UV-C 24 hours after transfection. Pretreatment with Astaxanthin was done 3 hours prior to irradiation, where indicated. Cells were harvested and luciferase activity was measured 14-18 hours following exposure. The ratio between Firefly and Renilla luciferase activity is represented as fold activation over the activity in unstimulated control cells. The standard error is based on two identical experiments.

### Experiments

The experiments were conducted in order to show that astaxanthin inhibits the activation of NF-κB effected by UV exposure and thus the expression of inflammatory cytokines and chemokines.

### MATERIALS AND METHODS

### Cell culture

The human fibroblast cell line Hela Tet/off was cultured in MEM alpha medium supplemented with 10% fetal calf serum, penicillin and streptomycin (Gibco Grand Island, NY). The day before transfection, cells were plated on 6 cm dishes in a medium containing 2.5% fetal calf serum. On the day of transfection, the cell culture medium was changed to fresh medium (2.5% fetal calf serum).

### Preparation of Astaxanthin

A stock solution of Astaxanthin was prepared by dissolving synthetic Astaxanthin (Sigma) in 99.6% ethanol. The concentration of Astaxanthin was measured by spectrophotometry, and the absorbance maximum at 474-479 nm was used to calculate the concentration, according to the formula: Absₘₐₓ/210.

### Plasmids

The 6xκB-Luc plasmid contains a firefly luciferase reporter gene driven by 6 NF-κB binding sites cloned upstream of a TK promoter (Meyer et al. 1993). The pRL-TK plasmid contains a Renilla luciferase reporter gene driven by the TK promoter and is used as a control for transfection efficiency (Promega).

### Transfection, UV irradiation and reporter gene analysis

Plasmid DNA (1µg of the 6xκB-Luc reporter gene plasmid and 200 ng of pRL-TK control plasmid) was added to a mixture of 4 µl fuGENE 6 transfection reagent (Boeringer Ingelheim, Germany) and 196 µl serum free medium and incubated for 15 min at room temperature, according to the manufacturers protocol. DNA/fuGENE 6 was added to HeLa cells at 50% confluency and the cells were left in a 37°C incubator. 24 hours after transfection, the medium was removed and cells were exposed to UV. For this, a Stratalinker 1800 (Stratagene) emitting a wavelength of 254 nm was used and 10 J/m² was applied. The same medium was then added back to the cells, and the cells were incubated at 37°C. Where indicated, cells were pretreated with Astaxanthin for 3 hours, by adding various concentrations of Astaxanthin to the medium. After addition, the Astaxanthin was left in the medium throughout the experiment.

Extracts were prepared 14-18 hours following exposure, and luciferase assays were performed using components of the luciferase assay system (Promega).

### Results

To analyse whether Astaxanthin has any effect on the activation of NF-κB we performed transient transfection experiments in HeLa cells. NF-κB has been shown to be responsive to UV-irradiation (reviewed in Pitette *et al*., 1997). Similarly, we demonstrate that exposure of the cells to UV-C induces expression of a transfected NF-κB-dependent reporter gene (Fig. 1). Moreover, pretreatment of the cells with Astaxanthin at the highest concentration repressed this activation almost 3-fold. These data indicate that Astaxanthin has an inhibitory effect on NF-κB, DNA binding activity of NF-κB and/or translocation of the NF-κB across the nuclear membrane by interfering with at the signalling components in the NF-κB activation pathway.

### REFERENCES

Rothwarf, D., M. and M. Karin (1999). The NF-κB activation pathway: A paradigm in information transfer from membrane to nucleus. Available at www.stke.org/cgi/content/full/OC_sigtrans; 1999/5/re1.

Baeuerle, P., A. and T. Henkel. (1994). Function and activation of NF-κB in the immune system. *Annu. Rev. Immunol.* **12**, 141-179.

Shakhov A., N., Collart, M., A., P., Vassalli, S., A., Nedospasov, and C., V. Jongeneel. (1990). κB-type enhancers are involved in lipopolysaccharide-mediated transcriptional activation of the tumor necrosis factor a gene in primary macrophages. *J. Exp. Med.* **171,** 35-47.

Libermann, T. A and D. Baltimore. (1990) Activation of interleukin-6 gene expression through the NF-κB trasncription factor. *Mol*. *Cell*. *Biol*. **10**, 237-2334.

Sha, W. ,C., Liou, H., C., Tuomanen, E., I. and D., T. Baltimore. (1995). Targeted disruption of the p50 subunit of NF-kappa B leads to multifocal defects in immune responses. *Cell*. **80**, 321-30.

Weih, F., Carrasco, D., Durham, S. K., Barton, D., S., Rizzo, C., A., Ryseck, R., P., Lira, S., A. and R. Bravo. (1995). Multiorgan inflammation and hematopoietic abnormalities in mice with a targeted disruption of Re1B, a member of the NF-kappa B/Rel family. *Cell* **80,** 331-40.

Köntgen, F., Grumont R., J., Strasser A., Metcalf, D., Li, R., Tarlinton, D. and S. Gerondakis. (1995). Mice lacking the c-rel proto-oncogene exhibit defects in lymphoid proliferation, humoral immunity, and interleukin-2-expression. *Genes Dev.* **9**, 1965-1977.

Neurath, M., F., Pettersson, S., Meyer zum Büschenfelde, K-H. and W. Strober. (1996). Local administration of antisense phosphorothioate oligonucleotides to the p65 subunit of NF-κB abrogates established experimental colitis in mice. *Nature Medicine.* **2**, 998-1004.

Piette, J., Piret, B., Bonizzi, G., Schoonbroodt, S., Merville, M-P., Legrand-Poels, S., and V. Bours (1997). Multiple redox regulation in NF-κB transcription factor activation. *Biol. Chem.* **378,** 1237-1245.

Meyer, M., Schreck, R., and P., A. Baeuerle. (1993). H2O2 and antioxidants have opposite effects on activation of NF-kappa B and AP-1 in intact cells: AP-1 as secondary antioxidant-responsive factor. *EMBO J.* **12**, 2005-2015.

## Claims

1. In vitro use of astaxanthin for pretreatment of cells prior to UV-exposure to inhibit the activation of NF-κB.

2. Use according to claim 1, wherein the astaxanthin is selected from the group consisting of astaxanthin from a natural source, synthetic astaxanthin and mixtures thereof.

3. Use according to claim 2, wherein the natural source is a culture of the alga *Haematococcus sp.*

## Patentansprüche

1. In-vitro-Verwendung von Astaxanthin zur Vorbehandlung von Zellen vor der UV-Bestrahlung zwecks Hemmung der Aktivierung von NF-kB.

2. Verwendung nach Anspruch 1, worin das Astaxanthin aus der Gruppe bestehend aus Astaxanthin aus einer natürlichen Quelle, synthetischem Astaxanthin und Mischungen daraus ausgewählt ist.

3. Verwendung nach Anspruch 2, worin die natürliche Quelle eine Kultur der Alge *Haematococcus sp*. ist.

## Revendications

1. Utilisation in vitro d'astaxanthine pour le prétraitement de cellules avant leur exposition aux rayons UV afin d'inhiber l'activation de NF-kB.

2. Utilisation selon la revendication 1, où l'astaxanthine est choisie parmi le groupe comprenant l'astaxanthine d'une source naturelle, l'astaxanthine synthétique et des mélanges ces dernières.

3. Utilisation selon la revendication 2, où la source naturelle est une culture de l'algue *Haematococcus sp.*
